# EUROPEAN PATENT SPECIFICATION

(11) **EP 2 349 090 B1**
(45) Date of publication and mention of the grant of the patent: **26.03.2014**
(21) Application number: 09741147.4
(22) Date of filing: 16.10.2009
(51) Int. Cl.: A61F 2/12

(54) **PROSTHETIC IMPLANT SHELL**
PROTHESENIMPLANTATSCHALE
ENVELOPPE D'IMPLANT PROTHÉTIQUE

(30) Priority: 17.10.2008 US 106458
(43) Date of publication of application: 03.08.2011
(60) Divisional application: 14155727.2
(73) Proprietor: Allergan, Inc., Irvine, CA 92612 (US)
(72) Inventor: YACOUB, Kerim, Solvang California 93463 (US); POWELL, Thomas E., Santa Barbara California 93111 (US)
(74) Representative: HOFFMANN EITLE
(86) International application number: PCT/US2009/061045
(87) International publication number: WO 2010/045581

(56) References cited:
- EP-A- 0 030 838
- DE-A1-102007 041 381
- US-A1- 2006 161 266

## Description

### Field of the Invention

The present invention relates to prosthetic implants, for example, mammary implants.

### Background

Implantable prostheses are commonly used to replace or augment body tissue. In the case of breast cancer, it is sometimes necessary to remove some or all of the mammary gland and surrounding tissue that creates a void that can be filled with an implantable prosthesis. The implant serves to support surrounding tissue and to maintain the appearance of the body. The restoration of the normal appearance of the body has an extremely beneficial psychological effect on post-operative patients, eliminating much of the shock and depression that often follows extensive surgical procedures. Implantable prostheses are also used more generally for restoring the normal appearance of soft tissue in various other areas of the body.

Fluid filled implants can be imaged and evaluated in vivo using mammography, magnetic resonance imaging (MRI), ultrasonography and computer tomography (CT). MRI is one of the most accurate imaging technologies available for breast implants. However, there remains a need for technologies which afford more accurate diagnostic imaging of fluid filled implants.

EP-A-0 030 838 discloses prosthetic implants comprising a silicone gel composition contained within a flexible container. The container is formed from a silicone rubber and is coated with a barrier layer containing a fluorinated organopolysiloxane, or the container is formed from a fluorosilicone rubber. The silicone rubber may contain an additive (e.g. titanium dioxide).

### Summary

The present invention provides a flexible shell for a fluid filled prosthetic implant which has enhanced visibility when viewed using conventional imaging technologies, for example, magnetic resonance imaging techniques. The implant may be a mammary implant.

Embodiments of the invention are as follows:
1. A flexible shell (22) for a fluid-filled prosthetic implant, the shell comprising at least one layer which comprises a matrix material and an additive having a higher density than the matrix material distributed in the matrix material;
   characterised in that:
   (i) the specific gravity of the shell is greater than about 1.20, and the additive is aluminium, brass, titanium, a titanium alloy, a nickel-titanium alloy, stainless steel or a mixture thereof; or
   (ii) the specific gravity of the shell is greater than about 1.20, the additive is titanium dioxide (TiO₂), and the shell includes an indicia (28) formed from fused titanium.
2. A shell according to 1, wherein the specific gravity of the shell is greater than about 1.40.
3. A shell according to 1, wherein the matrix material is a silicone elastomer.
4. A method for producing a flexible shell (22) for a fluid-filled prosthetic implant, the method comprising the steps of:
   providing a quantity of a matrix material in bulk form;
   combining the matrix material with a quantity of an additive in bulk form to produce a dispersion; and
   forming a shell from the dispersion, the shell comprising at least one layer which comprises the matrix material and the additive distributed in the matrix material;
   wherein:
      the additive has a higher density than the matrix material;
      the additive is aluminium, brass, titanium, a titanium alloy, a nickel-titanium alloy, stainless steel or a mixture thereof; and
      the specific gravity of the shell is greater than about 1.20.

The indicia may be in the form of a label indicating batch number, manufacturer, location of manufacture, model or style number, trademark, and/or other useful information relating to the implant. In one embodiment the indicia comprises a label in the form of a bar code, for example, etched by laser on an exterior surface of the shell.

### Brief Description of the Drawing

Features and advantages of the present invention will become appreciated as the same become better understood with reference to the specification, claims, and appended drawing of which:

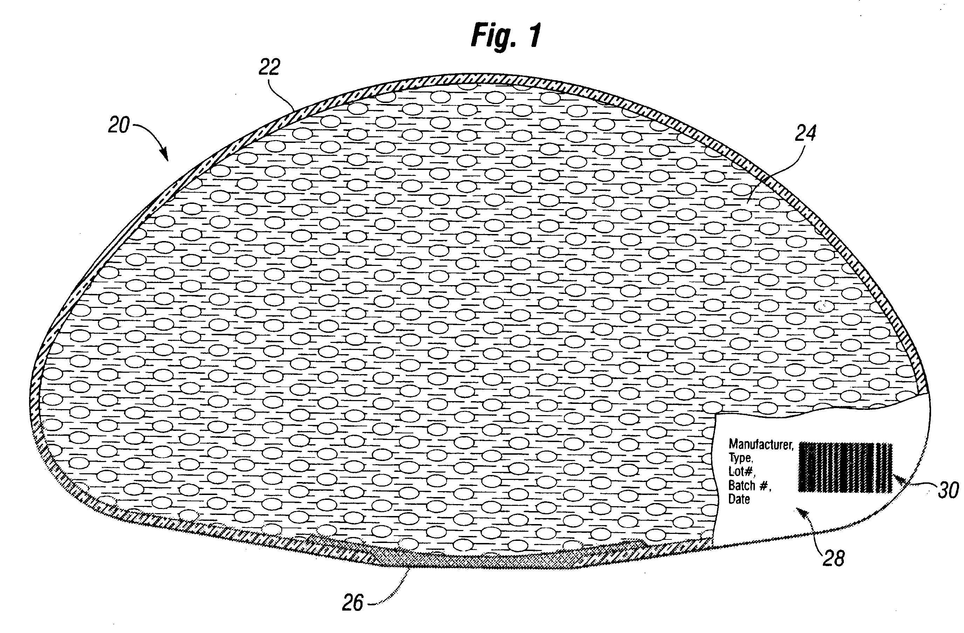

Figure 1 is a cross-sectional view through a fluid-filled prosthetic implant in accordance with an embodiment of the present invention.

### Detailed Description

The present application provides a flexible shell for a prosthetic implant. The implant may be a mammary implant useful for reconstruction or augmentation of the breast.

The matrix material is typically a silicone elastomer. The additive distributed within the matrix material causes the shell to have an increased density relative to an otherwise identical shell without the additive.

The shell of the present invention provides enhanced visibility, when imaged in vivo using conventional imaging techniques, relative to conventional silicone elastomer shells not including the additive. For example, the present shell and implants containing the shell are more readily visible in magnetic resonance images.

In one aspect of the invention, the shell is made up of a material having a density greater than the density of body tissue, for example, breast tissue, adjacent the implant when the implant has been implanted in a patient.

Specific gravity is defined as the ratio of the density of a given solid or liquid substance to the density of water at 4 °C (39°F). At this temperature, the density of pure water is about 1.0 g/ml (about 62.4 lb/ft³). Materials with a specific gravity greater than 1.0 have a higher density than pure water at 4 °C. For practical purposes, the density of a material in g/ml (or g/cc) is equivalent to the specific gravity of that material when water is used as the reference density.

Conventional silicone elastomer implant shells have a specific gravity close to that of water, and typically no greater than about 1.10 or about 1.15. In contrast, the shell of the present invention has a specific gravity greater than 1.20. The shell may have a specific gravity greater than about 1.40, greater than about 1.60, or greater than about 1.80.

The concentration of additive, for example, TiO₂. in the shell may be in the range of or between about 0.5% and about 25% by weight or between about 5% and about 10% by weight. In one embodiment, the concentration of TiO₂ in the shell is about 8% by weight.

The shell may comprise a single, unitary layer comprising the matrix material and additive as described elsewhere herein. In other embodiments, the implant shell comprises a plurality of such layers of material, wherein at least one of said layers comprises the matrix material and additive. In some embodiments of the invention, the shell has a tensile strength comparable to or greater than a tensile strength of an identical shell comprising the matrix material without said additive dispersed therein.

In one embodiment of the invention, the additive is added to a dispersion of the matrix material in the form of a paste or powder. The powder or paste may comprise the additive formulated with a resin, for example, a silicone fluid-resin. The desired concentration of additive in the final mixture may be calculated based on percent solids of silicone elastomer (rubber) in the batch of dispersion to the amount of additive added thereto. In a specific embodiment, an additive comprising TiO₂ is initially provided in a powder form and then is converted to a paste form by mixing said powder with a suitable polymer or other material. The additive "paste" is then combined with a liquid form of silicone elastomer including an appropriate solvent. The combining can be accomplished by mixing or other suitable technique to ensure substantially uniform distribution of the additive in the silicone elastomer. The liquid silicone elastomer/titanium dioxide dispersion is used to form the shells of the implants of the present invention, for example, using conventional dip-molding or rotational molding techniques known to those of skill in the art.

Fig. 1 illustrates a breast implant 20 comprising an exemplary shell of the present invention, the shell 22 comprising a matrix material and an additive distributed therein having a density greater than a density of the matrix material, such that the density of the shell material is greater than about 1.2 g/ml or greater than about 1.4 g/ml.

The implant 20 also comprises a fluid 24 enclosed by the shell 22. The fluid may be a silicone gel, saline or other appropriate prosthetic implant filler material. In some embodiments, the flush patch 26 covers a manufacturing hole formed on the shell during molding thereof. In other embodiments, the implant may include a fluid adjustment valve.

In one embodiment of the invention, the shell 22 includes marking, labeling or other indicia 28, formed on the shell 22 by contacting the shell with focused electromagnetic energy, for example, in the form of a laser, which causes discoloration of the additive component of the shell. In this embodiment, the additive is titanium dioxide and the indicia is formed of fused titanium.

For example, in one embodiment of the invention, the shell comprises a matrix material and an additive which facilitates marking of the shell, for example, when the shell is contacted with radiation, for example, ultraviolet ,visible, or near infrared radiation, or other radiation form which will react with the additive to form the indicia without compromising the integrity (e.g. strength) of the shell. Such energy source can be supplied by a conventional laser source. For example, in one embodiment of the invention, the additive comprises titanium dioxide and the shell includes marking, labeling or other indicia 28 of titanium, formed on the shell by reaction of focused energy with the titanium dioxide component of the shell. In one embodiment, the indicia is a computer-readable marking, for example, in the form of a bar code which provides information about the shell.

Marking of the shell may be achieved by moving a laser beam over a portion of the shell using conventional beam steering methods, by moving the shell in relation to the laser beam and/or by masking the shell and applying light energy to an unmasked portion of the shell.

Suitable lasers for use in accordance with the present invention include, for example, neodymium:yttrium aluminum garnet (Nd:YAG) lasers, carbon dioxide(CO₂) lasers, diode lasers and excimer lasers.

Conventional YAG lasers emit light in the near-infrared spectrum at wavelengths of 1064 nm. Such lasers typically have continuous power outputs of from about 1 watt to about 50 watts, and can be operated in a pulsed mode at typical peak powers of from about 1 watt to about 45 kilowatts. For pulsed mode operation, frequencies of from about 1 to about 64,000 pulses/second may be used.

Suitable lasers for marking the shells of the present invention include EPILOG Legend Model 6000 L24EX-30W, EPILOG Helix Model 8000. Suitable software for use with this equipment includes CADLink Engravelab 5.0 software. Another suitable laser is Electrolox Razor 30 W razor with Scriba3 software which can interact with Oracle and generate data for the marking of the shell.

In accordance with one embodiment of the present invention, the size of the laser spot that impinges the shell may have a diameter of between about 0.1 micron to about 500 microns, or greater.

It will be appreciated that the laser parameters may be controlled in order to provide sufficient localized radiation to create titanium-based marking from the titanium dioxide in the shell while avoiding damage to the integrity of the shell.

In some embodiments movement of the laser beam is controlled by a computer which may be used to create the indicia.

The shell may contain an additive besides titanium dioxide, for example, another suitable metal oxide, that is biocompatible and reacts with focused energy applied to the shell containing the additive to produce visible marking on the shell.

In one embodiment, the laser-inscribed indicia are detectable on the shell in vivo, for example, using MRI or other suitable imaging technique. For example, on an MRI image, the implant itself will be visible and, in addition, the indicia thereon will also be detectable, for example, distinctly visible and/or otherwise readable.

Alternatively, or in addition, the same information may be incorporated into a computer readable bar code 30 that makes automatic identification though a scanner possible. The inclusion of a non-ferromagnetic or weakly ferromagnetic metal such as TiO₂ in the shell 22 enhances the visibility of such a label, as the metal at the surface fuses to create visible lines without weakening the shell material.

## Claims

1. A flexible shell (22) for a fluid-filled prosthetic implant, the shell comprising at least one layer which comprises a matrix material and, distributed in the matrix material, an additive having a higher density than the matrix material;
**characterised in that**:
(i) the specific gravity of the shell is greater than about 1.20, and the additive is aluminium, brass, titanium, a titanium alloy, a nickel-titanium alloy, stainless steel or a mixture thereof; or
(ii) the specific gravity of the shell is greater than about 1.20, the additive is titanium dioxide (TiO₂), and the shell includes an indicia (28) formed from fused titanium.

2. A shell according to Claim 1, wherein the specific gravity of the shell is greater than about 1.40.

3. A shell according to Claim 1, wherein the matrix material is a silicone elastomer.

4. A method for producing a flexible shell (22) for a fluid-filled prosthetic implant, the method comprising the steps of:
providing a quantity of a matrix material in bulk form;
combining the matrix material with a quantity of an additive in bulk form to produce a dispersion, the additive having a higher density than the matrix material; and
forming a shell from the dispersion, the shell comprising at least one layer which comprises the matrix material and the additive distributed in the matrix material;
**characterised in that**:
the additive is aluminium, brass, titanium, a titanium alloy, a nickel-titanium alloy, stainless steel or a mixture thereof; and
the specific gravity of the shell is greater than about 1.20.

## Patentansprüche

1. Flexible Umhüllung (22) für ein mit einem Fluid gefülltes Protheseimplantat, wobei die Umhüllung mindestens eine Schicht umfasst, die ein Matrixmaterial und im Matrixmaterial verteilt ein Additiv umfasst, das eine höhere Dichte als das Matrixmaterial aufweist,
**dadurch gekennzeichnet, dass**:
(i) das spezifische Gewicht der Umhüllung größer als etwa 1,20 ist, und das Additiv Aluminium, Messing, Titan, eine Titanlegierung, eine Nickel-TitanLegierung, rostfreier Stahl oder eine Mischung daraus ist; oder
(ii) das spezifische Gewicht der Umhüllung größer als etwa 1,20 ist, das Additiv Titandioxid (TiO₂) ist, und die Umhüllung eine aus verschmolzenem Titan gebildete Kennzeichnung (28) einschließt.

2. Umhüllung gemäß Anspruch 1, worin das spezifische Gewicht der Umhüllung größer als etwa 1,40 ist.

3. Umhüllung gemäß Anspruch 1, worin das Matrixmaterial ein Silikonelastomer ist.

4. Verfahren zur Herstellung einer flexiblen Umhüllung (22) für ein mit einem Fluid gefülltes Protheseimplantat, wobei das Verfahren die Schritte umfasst:
Bereitstellen einer Menge eines Matrixmaterials in Bulk-Form;
Kombinieren des Matrixmaterials mit einer Menge eines Additivs in Bulk-Form, um eine Dispersion zu erzeugen,
wobei das Additiv eine höhere Dichte als das Matrixmaterial hat; und
Bilden einer Umhüllung aus der Dispersion, wobei die Umhüllung mindestens eine Schicht umfasst, die das Matrixmaterial und das im Matrixmaterial verteilte Additiv umfasst;
**dadurch gekennzeichnet, dass**:
das Additiv Aluminium, Messing, Titan, eine Titanlegierung, eine Nickel-Titan-Legierung, rostfreier Stahl oder eine Mischung daraus ist; und
das spezifische Gewicht der Umhüllung größer als etwa 1,20 ist.

## Revendications

1. Enveloppe flexible (22) pour un implant prosthétique rempli de fluide, l'enveloppe comprenant au moins une couche qui comprend un matériau de matrice et, distribué dans le matériau de matrice, un additif ayant une masse volumique plus élevée que le matériau de matrice ;
**caractérisée en ce que** :
(i) la densité de l'enveloppe est supérieure à environ 1,20, et l'additif est l'aluminium, le laiton, le titane, un alliage de titane, un alliage de nickel-titane, l'acier inoxydable ou un mélange de ceux-ci ; ou
(ii) la densité de l'enveloppe est supérieure à environ 1,20, l'additif est le dioxyde de titane (TiO₂), et l'enveloppe comprend un indice (28) formé de titane fondu.

2. Enveloppe selon la revendication 1, dans laquelle la densité de l'enveloppe est supérieure à environ 1,40.

3. Enveloppe selon la revendication 1, dans laquelle le matériau de matrice est un élastomère de silicone.

4. Procédé pour produire une enveloppe flexible (22) pour un implant prosthétique rempli de fluide, le procédé comprenant les étapes de :
fourniture d'une quantité d'un matériau de matrice sous forme de vrac ;
combinaison du matériau de matrice avec une quantité d'un additif sous forme de vrac pour produire une dispersion, l'additif ayant une masse volumique plus élevée que le matériau de matrice ; et
formation d'une enveloppe à partir de la dispersion, l'enveloppe comprenant au moins une couche qui comprend le matériau de matrice et l'additif distribué dans le matériau de matrice ;
**caractérisé en ce que** :
l'additif est l'aluminium, le laiton, le titane, un alliage de titane, un alliage de nickel-titane, l'acier inoxydable ou un mélange de ceux-ci ; et
la densité de l'enveloppe est supérieure à environ 1,20.
